(19) **Europäisches Patentamt European Patent Office Office européen des brevets**

(11) **EP 4 217 741 B1**

(12) **EUROPEAN PATENT SPECIFICATION**

(45) Date of publication and mention
of the grant of the patent:
**16.04.2025 Bulletin 2025/16**

(21) Application number: **21778501.3**

(22) Date of filing: **22.09.2021**

(51) International Patent Classification (IPC):
**G01N 33/543** (2006.01)  **G01N 33/58** (2006.01)
**G01N 27/00** (2006.01)

(52) Cooperative Patent Classification (CPC):
**G01N 33/5438; G01N 33/587;** G01N 27/3277

(86) International application number:
**PCT/EP2021/076100**

(87) International publication number:
**WO 2022/063847 (31.03.2022 Gazette 2022/13)**

(54) **METHOD FOR DETECTING AN ANALYTE WITH THE HELP OF METAL NANOPARTICLES ON AN ELECTRODE**

VERFAHREN ZUM NACHWEIS EINES ANALYTEN MIT DER HILFE VON
METALLNANOPARTIKELN AUF EINER ELEKTRODE

PROCEDE DE DETECTION D'UN ANALYTE A L'AIDE DE NANOPARTICULES METALLIQUES SUR
UNE ELECTRODE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.09.2020 EP 20197810**

(43) Date of publication of application:
**02.08.2023 Bulletin 2023/31**

(73) Proprietors:
• **Roche Diagnostics GmbH
68305 Mannheim (DE)**
Designated Contracting States:
**DE**
• **F. Hoffmann-La Roche AG
4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**

(72) Inventors:
• **BAEUMNER, Antje
93053 Regensburg (DE)**
• **BECK, Franziska
68305 Mannheim (DE)**

• **HORN, Carina
68305 Mannheim (DE)**

(74) Representative: **Altmann Stößel Dick
Patentanwälte PartG mbB
Theodor-Heuss-Anlage 2
68165 Mannheim (DE)**

(56) References cited:
• **YEKATERINA KHRISTUNOVA ET AL:
"Preparation and Investigation of Silver
Nanoparticle-Antibody Bioconjugates for
Electrochemical Immunoassay of Tick-Borne
Encephalitis", SENSORS, vol. 19, no. 9, 7 May
2019 (2019-05-07), pages 2103, XP055771934,
DOI: 10.3390/s19092103**
• **NICOLE E. POLLOK ET AL: "Electrochemical
Detection of NT-proBNP Using a
Metalloimmunoassay on a Paper Electrode
Platform", ACS SENSORS, vol. 5, no. 3, 10 March
2020 (2020-03-10), pages 853 - 860,
XP055772046, ISSN: 2379-3694, DOI: 10.1021/
acssensors.0c00167**

- NAN HAO ET AL: "An electrochemical immunosensing method based on silver nanoparticles", JOURNAL OF ELECTROANALYTICAL CHEMISTRY, ELSEVIER, AMSTERDAM, NL, vol. 656, no. 1, 24 January 2011 (2011-01-24), pages 50 - 54, XP028208032, ISSN: 1572-6657, [retrieved on 20110213], DOI: 10.1016/J.JELECHEM.2011.01.029

- MATEUSZ S. SZYMANSKI ET AL: "Preparation and quality control of silver nanoparticle-antibody conjugate for use in electrochemical immunoassays", JOURNAL OF IMMUNOLOGICAL METHODS., vol. 387, no. 1-2, 12 November 2012 (2012-11-12), NL, pages 262 - 269, XP055494421, ISSN: 0022-1759, DOI: 10.1016/j.jim.2012.11.003

## Description

The present invention relates to a method for detecting at least one analyte by electrochemical detection, a device for detecting at least one analyte in a sample by electrochemical detection. The method of the present invention comprises the following steps: contacting a fluid sample suspected to comprise the at least one analyte with the surface of an electrode comprising at least one binding agent capable of binding to the analyte; contacting the fluid sample suspected to comprise the at least one analyte with a least one detection agent, wherein the at least one detection agent comprises at least a further binding agent capable of binding to the analyte and a label, wherein the label comprises a metal nanoparticle with a standard redox potential E° between 0 V and 1.2 V; forming a detection complex on the surface of the electrode comprising at least the at least one binding agent, the at least one detection agent and the analyte; precipitating at least a part of the label onto the electrode surface; and detecting the at least one analyte by electrochemical detection; wherein the method comprises a step of dissolving at least a part of the label by oxidation prior to precipitating at least a part of the label, and wherein the step of dissolving at least a part of the label by oxidation comprises applying a voltage at the electrode suitable to oxidize the metal nanoparticle, wherein a positive potential higher than the oxidation potential of the respective metal nanoparticle is applied as this voltage, wherein the applied voltage results in direct oxidation of the metal nanoparticle.

Further, the present invention provides a device for detecting at least one analyte by electrochemical detection, comprising at least one working electrode, wherein the surface of the working electrode comprises at least a linker for coupling the at least one binding agent, and wherein the working electrode is configured to allow for the formation of a detection complex comprising at least one detection agent, the at least one binding agent and the analyte on the surface of the electrode exposable to a fluid sample, wherein the at least one detection agent comprises at least one further binding agent and a label, wherein the label comprises a metal nanoparticle with a standard redox potential E° between 0 V and 1.2 V; and operatively linked thereto an analyzing unit configured for applying a voltage at the electrode suitable to oxidize the metal nanoparticle, and configured for measuring the detectable signal produced by the detection agent when detecting the at least one analyte.

## Background art

Recent advances in modern medicine present new challenges for immunoassay based analyte detection and analyte sensors. A fast detection of certain biomarkers in an ultra-low concentration range is crucial for early diagnosis, which helps to increase patient survival rates. The use of low sample volumes of biological fluids like blood is desirable to accelerate the testing procedures. Moreover, a reliable target quantification should be striven for assessing the severity of the disease and adapting the medication appropriately. Simultaneously, reproducibility, selectivity, easy handling and low-cost of the device have to be preserved.

Electrochemical detection methods are very well suited for these purposes. In electrochemical detection, a biological recognition element is usually coupled to an electrical transducer, which translates the binding event into an electrical signal. This creates a rapid and simple detection method. The instrumentation is rather inexpensive and can be easily miniaturized towards a portable point-of-care device. Commonly, the measurements are performed in an amperometric or potentiometric fashion, amperometric biosensors often being more attractive due to their good sensitivity, accuracy and wide linear range. Another advantage of electrochemical detection over e.g. optical sensors is that the measurements can be performed in small volumes (few microliters) and eventually in turbid and colored samples without the need of prior purification. In immunoassays radioactive, fluorescent, bio- and chemiluminescent probes or enzymes are usually used as markers. Starting from the 1970s the use of metal-based labels in so-called "metalloimmunoassays" is reported overcoming disadvantages of formerly used common markers.

The use of metallic nanoparticles (mNPs) with a size between 10 and 50 nm for example got increasing attention over the past years. The physical, electrical and optical properties are highly different from those of the bulk metal and can be tailored by synthesis, chemical and/or biological modifications. Due to the high surface-to-volume ratio, mNPs are able to catalyze reactions and accelerate the electron transfer rate efficiently (Hao et al., 2011).

Using nanoparticles in an electrochemical biosensor may increase the loading of the electrode with electroactive species drastically compared to single molecule labels. This leads to an enormous amplification of current signal (Ting et al., 2009). Amplification power of mNPs is similar to the best enzyme labels, but mNP labels do not require timed signal recording and are not prone to denaturing during storage. Moreover, electrical biosensors using mNPs show high multiplexing capabilities due to the diversity of modifications and metals which could be used.

Most often reported metal nanoparticle labels are gold nanoparticles (AuNPs) due to their unique optical, catalytic and electronic properties. Their excellent biocompatibility renders them highly useful for immunocytochemistry and cell biology. Moreover, they can easily be synthesized and modified and show great colloidal stability. Most assays exploit their optical properties, but in the 2000s also electrochemical immune- and DNA sensing gained increasing attention. First procedures included a chemical dissolution of gold in a $HBr/Br_2$ solution, an accumulation on the electrode and stripping analysis. These approaches proved to be very sensitive. However, using this highly toxic solution is not favorable and supplementary steps are always time consuming and prone to errors.

Therefore, alternative approaches with direct electrochemical dissolution were developed. Pumera et al., for example, applied a three-step analysis consisting of adsorption of the AuNPs onto the electrode surface, oxidative dissolution in presence of HCl and reverse electro-reduction. Most electrochemical assays reported in literature using AuNPs as label are used for the quantification of DNA. Immunoassays are more challenging due to the higher complexity of proteins, the absence of amplification technologies like PCR and the stronger non-specific binding to solid supports. A combination of gold and silver has been described in the beginning of the 2000s.

Although silver has a greater electrochemical activity that leads to well-defined, sharp reduction peaks and the dissolution of the silver nanoparticles (AgNPs) is easier than for gold, at present, most electrochemical analyses using AgNP tags to detect DNA. In 2010, a protein quantification assay has been described (Szymanski et al., 2010).

Researchers have managed to adsorb the antigen and use AgNP-labeled antibodies for detection (Hao et al., 2011). Alternatively, AgNPs were detached chemically and pipetted separately onto an electrode for subsequent quantification, when applied in a sandwich approach (Szymanski et al., 2010). Pollok et al. describe a metalloimmunoassay using silver nanoparticles, wherein a sandwich complex is formed in the wells of a microtiter plate and then transferred to a paper-based electrode for subsequent electrochemical detection. Khristunova et al. (2019) developed and tested a new simple electrochemical immunosensor approach for the determination of antibodies to tick-borne encephalitis virus (TBEV) in immunological products. The approach involves a step of preliminary dissolution and oxidation of Ag in $HNO_3$ followed by a step of detecting the silver reduction signal in the bioconjugates with antibodies (Ab@AgNP). The signal is read by cathodic linear sweep voltammetry (CLSV) through the detection of silver chloride reduction on a gold-carbon composite electrode (GCCE).

However, up to date, no one-step immunoassay on an electrode surface with a direct electrochemical detection utilizing AgNPs has been described.

Problem to be solved

Hence, there is still an unmet need for simple, cost-efficient but still reliable and highly sensitive analyte detection methods and means, in particular detection methods and means that are suitable for point-of-care devices or fluidic devices.

Summary

This problem is addressed by a method and a device for detecting at least one analyte by electrochemical detection with the features of the independent claims. Advantageous embodiments which might be realized in an isolated fashion or in any arbitrary combinations are listed in the dependent claims.

Accordingly, the present invention relates to a method for detecting at least one analyte by electrochemical detection, comprising:

(i) contacting a fluid sample suspected to comprise the at least one analyte with the surface of an electrode comprising at least one binding agent;
(ii) contacting the fluid sample suspected to comprise the at least one analyte with a least one detection agent, wherein the detection agent comprises at least a further binding agent and a label, wherein the label comprises a metal nanoparticle with a standard redox potential E° between 0 V and 1.2 V, with a standard redox potential E° between 0 V and 1.2 V.
(iii) forming a detection complex on the surface of the electrode comprising at least the at least one binding agent, the detection agent and the analyte;
(iv) precipitating at least a part of the label onto the electrode surface; and
(v) detecting the at least one analyte by electrochemical detection;

wherein the method comprises a step of dissolving at least a part of the label by oxidation prior to precipitating at least a part of the label in step (iv), and
wherein the step of dissolving at least a part of the label by oxidation comprises applying a voltage at the electrode suitable to oxidize the metal nanoparticle, wherein a positive potential higher than the oxidation potential of the respective metal nanoparticle is applied as this voltage, wherein the applied voltage results in direct oxidation of the metal nanoparticle.

The present method has the advantage that the nanoparticles, due to their electrochemical activity, provide a highly sensitive way of analyte detection. Further, the method provides a simple analyte detection means and is adaptable to a point-of-care setting as no further addition of any solution is necessary once it is used in a biological sample. The method may be performed as a one-step immunoassay on the electrode surface, which provides a simple and fast way of detection. Moreover, the inventors have found that the detection agent has excellent long-term storage stability.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

The term "analyte" as used herein, relates to a compound, specifically to a chemical molecule, more specifically to an organic molecule, even more specifically to a biological macromolecule such as for example a carbohydrate, a lipid, a polynucleotide or a polypeptide. In particular, the analyte may be a protein, a carbohydrate or a lipid, more particularly a protein. More particularly, the term "analyte" may relate to a protein present in a body fluid of a mammalian species including human, even more particularly a blood protein. "Ana-lytes" according to the present invention in particular include human blood proteins such as proBNP, NT-proBNP, immunoglobulins, TSH, Troponin T or Tropinin I, D-Dimer, b-HCG, bacterial or viral proteins and/or nucleic acids.

As used herein, the term "body fluid" relates to all bodily fluids of a subject known to comprise or suspected to comprise the analyte of the present invention, including blood, plasma, serum, lacrimal fluid, urine, lymph, cerebrospinal fluid, bile, stool, sweat, and saliva. Preferably, the body fluid is blood.

The term "detecting" or "detection" relates to the quantification of the amount of analyte present in a sample, in particular a sample of a body fluid. The term "detecting" or "detection" specifically relates to measuring the amount or concentration of said analyte, preferably semi-quantitatively or quantitatively. The detection of the amount of the analyte can be accomplished in a variety of ways known to the skilled person or detailed herein below. In accordance with the present invention, detecting the amount of the analyte can specifically be achieved by electrochemical detection; more specifically electrochemical detection comprising a pulse voltammetry measurement, even more specifically comprising a differential pulse voltammetry (DPV) measurement. Moreover, the "detecting" may involve the formation of a detection complex comprising the at least one detection agent, the at least one binding agent and the analyte on the surface of the electrode.

The term "detection complex", as used herein, may refer to a complex comprising or consisting of the detection agent, the binding agent and the analyte. Moreover, it may refer to a complex comprising or consisting of the analyte complex as specified below and the detection agent.

The detection complex according to the present invention may be formed on the surface of the electrode in particular on the surface that has been exposed to the fluid sample or is exposable to the fluid sample. The detection complex may typically form upon binding of the analyte to the binding agent and the detection agent.

Typically, the binding agent is immobilized or is configured to become immobilized on the surface of the electrode exposable to the fluid sample, more typically the binding agent is immobilized or is configured to become immobilized on the surface of the electrode exposable to the fluid sample via a linker. In particular, the linker couples the binding agent to the surface of the electrode by covalent or reversible linkage to achieve immobilization; more particularly by reversible linkage. Suitable linkers used according to the present invention may include: streptavidin, avidin, biotin, neutravidin, polystrepatavidin and related compounds, and chemical linkers like maleimide, pyrene butyric acid, digoxigenin/anti-digoxigenin system, FITC/anti-FITC system, nickel-poly histidine system and the like. However, the binding agent may in a particular embodiment be immobilized or configured to become immobilized on the surface of the electrode exposable to the fluid sample without a linker for example the binding agent may be immobilized by adsorption onto the electrode surface.

The term "amount" as used herein encompasses the absolute amount of the analyte referred to herein, the relative amount or concentration of the analyte referred to herein as well as any value or parameter that correlates thereto. Such values or parameters comprise intensity signal values from all specific physical or chemical properties obtained from the analyte referred to herein by measurements. It is to be understood that values correlating to the aforementioned amounts or parameters can also be obtained by all standard mathematical operations.

The term "electrochemical detection" as used herein, refers to detecting an analyte by electrochemical means. Electrochemical means may include amperometric and/or potentiometric measurements using for example at least a working electrode, a reference electrode and/or a counter electrode. In particular, according to the present invention the term "electrochemical detection" may refer to the detection of labels having an electrochemically detectable property. Specifically, electrochemical detection according to the present invention involves pulse voltammetry, more specifically differential pulse voltammetry (DPV).

The term "sample" as used herein, relates to a sample of a fluid, in particular a body fluid, a sample from a tissue or an organ, or a sample of wash/rinse fluid or a swab or smear obtained from an outer or inner body surface. In particular, the sample is suspected to comprise an analyte as specified herein, or the sample, specifically comprises at least one analyte as specified elsewhere herein. In particular, the sample may be a fluid sample obtained from, for example, a body fluid such as blood, plasma, serum, urine, saliva, or lacrimal fluid sample. Samples typically can be obtained by use of brushes, (cotton) swabs, spatula, rinse/wash fluids, punch biopsy devices, puncture of cavities with needles or lancets, or by surgical instrumentation. However, samples obtained by well-known techniques including, in particular, scrapes, swabs or biopsies from the urogenital tract, perianal regions, anal canal, the oral cavity, the upper aerodigestive tract and the epidermis may also be included as samples of the present invention. Fluid samples may be obtainable by dissolving, suspending or dispersing a sample in an appropriate liquid including water as known by the skilled artisan.

The terms "binding agent" and the term "further binding agent" used according to the present invention may both refer to any kind of chemical moiety capable of binding the analyte. The binding agent and the further binding agent in particular may be selected from antibodies and fragments thereof, nucleic acids, aptamers, peptide nucleic acids (PNAs), receptor or ligand proteins or peptides, and enzymes, and or mixtures thereof; preferably the binding agent and/or the further binding agent comprises an antibody, more preferably an IgG. The terms "binding agent" and "further binding agent" may refer to identical or to different chemical moieties. In accordance with the present invention the "binding agent" may be understood to be immobilized or configured to become immobilized on the surface of electrode while the "further binding agent" is comprised in the detection agent. Typically, the binding agent may comprise a linker as specified herein above, even more typically the binding agent may be biotinylated. Alternatively, the binding agent may become immobilized on the surface of the electrode using chemical linkers as specified herein above. Typically, the further binding agent may be attached to the label. The attachment may more typically comprise unspecific binding of the metal atoms in the label over thiol- and/or amine groups to the further binding agent. Even more typically the attachment of the further binding agent to the label may be irreversible.

The binding agent and/or the further binding agent may preferably comprise or consist of an antibody or antibody fragment capable of binding the analyte, e.g. an IgG. Suitable antibodies or antibody fragments capable of binding the analyte are commercially available and/or can be produced by methods known to the person skilled in the art. The binding agent and/or the further binding agent may more preferably have a binding affinity in the nanomolar range.

A suitable binding agent used according to the present invention may be a primary antibody also referred to as capture antibody herein, e.g. an IgG. A primary antibody or capture antibody is an antibody that binds directly to the analyte. The term "direct binding" typically is understood as a direct intermolecular interaction between the binding agent and the analyte. More typically, "direct binding" relates to the formation of non-covalent bonds or non-covalent interactions between the binding agent and the analyte such as van der Waals forces, dipole-dipole interactions, ion-dipole interactions, hydrogen bonds, electrostatic interactions, pi-stacking. Even more typically, "direct binding" refers to the interaction of the binding agent, e.g. an antibody, with the analyte, e.g. an antigen.

The term "detection agent" relates to a species used according to the present invention to produce a detectable signal correlating with the amount of the analyte in the sample. In particular, the detection agent comprises at least or consists of the further binding agent and a label, wherein the label comprises or consist of a metal nanoparticle, or a nanoparticle comprising organic mediators like nitrosoanilines, phenazium derivatives or ferrocene with a standard redox potential $E°$ between 0 V and 1.2 V; in particular a metal nanoparticle with a standard redox potential $E°$ between 0 V and 1.2 V. A typical detection agent used according to the present invention comprises an IgG antibody or fragment thereof and/or a metal nanoparticle. More typically, the detection agent comprises an IgG antibody or fragment thereof attached to a metal nanoparticle, for example by unspecific binding of thiol and/or amine groups of the antibody or fragment thereof to the metal atoms.

The metal nanoparticle has a redox potential between 0 V and 1.2 V; typically, the metal nanoparticle comprises or is a silver nanoparticle. Even more typically, the IgG of the binding agent and the IgG of the detection agent may recognize the same antigen, i.e. the same analyte.

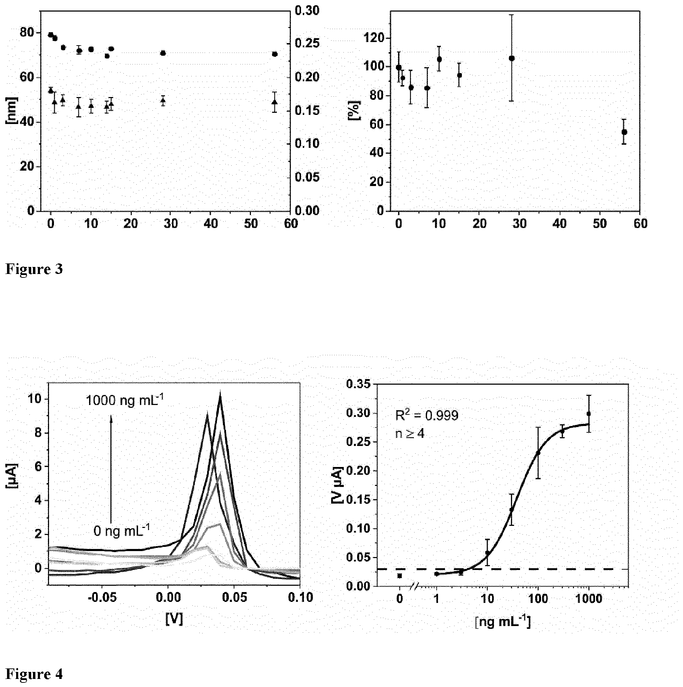

The detection agent may additionally comprise a blocking agent. The term "blocking agent" may typically refer to any substance capable of binding non-specifically to the detection agent and/or the label and/or any remaining metallic surface; in particular the blocking agent may comprise or consist of a polypeptide such as bovine serum albumin (BSA) or any other polypeptide capable of binding non-specifically to the detection agent and/or the label and/or any remaining metallic surface. The blocking advantageously may avoid adsorption of other sample components (e.g. blood proteins) to the nanoparticle and adsorption of the nanoparticle to the electrode surface. This can result in a positive effect on the long-term stability (Fig. 3) of the detection agent and an analyte sensor comprising such a detection agent.

According to the present invention, the "detectable signal" refers to an electrochemical signal such as commonly produced by electrochemical detection; such signals include e.g. a change in the current measured between working electrode and counter electrode depending on the amount of the analyte in the sample. In the electrochemical measurements used in the methods of the invention, typically a potential is applied between working electrode and counter electrode. This potential may in particular be monitored to be at the appropriate value for the working electrode between working electrode and reference electrode. More particularly, the current may be measured between working electrode and counter electrode. The term "label", as used herein, relates to a compound adapted for making the presence of a molecule or complex comprising said label detectable. Typically, the label has a detectable property, typically an electrochemical, optical or/and enzymatic property. In particular, the label used according to the present invention has an electrochemically detectable property. As will be understood, the parameter determined to detect said detectable property will also be referred to as "signal" or "detectable signal". For the avoidance of doubt, it is noted that a signal may also be detected to be zero or below a detection limit.

According to the present invention, the label may be irreversibly, preferably covalently, attached to the at least one further binding agent. However, unspecific attachment between the label and the further binding agent, in particular between the metal atoms and the further binding agent, more particularly between the metal atoms and thiol and amine groups of the further binding agent is also suitable and may be preferred. Methods of attaching the label to the further binding agent are known to the skilled artisan and include attachment via a linker and unspecific attachment as specified above. Suitable linkers are specified elsewhere herein.

According to the present invention, the label comprises or consists of a metal nanoparticle. The term "metal nanoparticle" refers to metal particles that have a particle size within the range of several nanometers, typically a particle size of 1 to 300 nm, more typically a particle size between 5 and 200 nm; even more particularly a particle size between 10 and 100 nm. Appropriate ways of determining the particle size of the nanoparticle are known to the person skilled in the art. Suitable metal nanoparticles according to the present invention include those that have a standard redox potential E° of between 0 V and 1,2 V, in particular those that have a standard redox potential E° of between 0.4 V and 1,0 V, more particularly a standard redox potential E° of about 0.8 V. Alternatively, organic materials and hybrid materials that have a redox potential of said range may be suitable as a label, too, as mentioned elsewhere herein. The standard redox potential $E^0$ may be determined using common methods known to the skilled artisan. Typically, the standard reduction potential ($E^0$) may be measured under standard conditions: 25 °C, an activity for each ion participating in the reaction of 1, a partial pressure of 1 bar (1013 hPa) for each gas that may be part of the reaction, and metals in their pure state. The standard redox potential is typically defined relative to a standard hydrogen electrode (SHE) reference electrode, which may be arbitrarily given a potential of 0.00 V.

Specifically, metal nanoparticles used according to the present invention include nanoparticles of silver, or alloys thereof; more specifically silver nanoparticles, or nanoparticles comprising or consisting of an silver/gold alloy. Even more specifically, the label used according to the present invention may comprise or may consist of a silver nanoparticle. Even more specifically, the label used according to the present invention may be a modified silver nanoparticle, preferably a citrate-capped or citrate-stabilized silver nanoparticle. Suitable nanoparticles are commercially available e.g. from nanoComposix (www.nanocomposix.com).

Using the labels according to the present invention, may offer the advantage of combining a highly sensitive electrochemical detection with a highly specific immunological binding, e.g. an antibody based sandwich binding, approach.

The method according to the present invention comprises step (i) of contacting the fluid sample suspected to comprise the at least one analyte with the surface of an electrode comprising at least one binding agent capable of binding to the analyte. Typically the at least one binding agent is immobilized on the surface of the electrode or is configured to become immobilized on the surface of the electrode. This has the advantage that it may allow for the forming of the detection complex comprising the binding agent, the detection agent and the analyte in step (iii) at a given position, i.e. on the surface of the electrode in a single step, i.e. in a one-step reaction. This simplifies the way of detection and makes the method particularly suitable for use in point of care devices. As a further advantage, in the method of the invention, the detection complex may be immobilized on the surface of the electrode, which may increase or even maximize the amount of the analyte available for the electrochemical detection using the electrode. This may increase the sensitivity of the detection.

The method according to the invention may further comprise an additional step of blocking the surface of the electrode exposable to the fluid sample. Typically, the step of blocking comprises contacting said surface with a blocking agent, capable of binding non-specifically to said surface. Suitable blocking agents are known to the skilled artisan. A particularly suitable blocking agent may comprise or consist of a polypeptide and/or non-ionic surfactants such as Tween 20, and/or mixtures thereof. A particularly suitable blocking agent may comprise or consist of a peptide or protein e.g. BSA or casein. The term "blocking" refers to non-specifically binding to any available binding site on said surface, in particular to any binding sites of the binding agent when immobilized on said surface. Advantageously, the blocking agent may contribute to the stabilization of the immobilized binding agent, especially for dry storage.

"Contacting" of the fluid sample with the surface of an electrode comprising at least one binding agent and/or with the at least one detection agent may take place by means and methods known to the skilled artisan. In particular by mixing the fluid sample with the detection agent, by adding the detection agent to the fluid sample in any suitable way, or by letting the fluid sample or a mixture comprising the fluid sample flow over the electrode surface. The step (i) of "contacting a fluid sample suspected to comprise the at least one analyte with the surface of an electrode comprising at least one binding agent capable of binding to the analyte" is advantageous as the binding of the analyte to the binding agent may occur directly on the surface of the electrode where the formation of the detection complex and the detection may take place. Hence, advantageously no transfer of the analyte bound to the binding agent and/or of the detection complex is necessary.

The method according to the present invention may comprise a step of forming an analyte complex on the surface of the electrode comprising the binding agent and the analyte, prior or simultaneously to step (iii) of forming of the detection complex. Also, the analyte complex may be immobilized on the surface of the electrode due immobilization of the binding agent on the surface of the electrode.

Contacting the analyte complex with at least one detection agent, wherein the detection agent comprises at least a further binding agent and a label, wherein the label comprises a metal nanoparticle, in step (ii) may be performed simultaneously with step (i) or thereafter. Alternatively, the detection agent may be contacted with and/or added to the fluid sample suspected to comprise the at least one analyte prior to contacting the fluid sample with the surface of an electrode comprising the at least one binding agent in step (i).

The at least one binding agent may be immobilized on the surface of the electrode prior to contacting the electrode with the fluid sample or the at least one binding agent may be contacted with the fluid sample prior to immobilizing the binding agent on the surface of the electrode. Hence, "comprising at least one binding agent" as specified in step (i) may refer to the binding agent being present in the fluid sample and/or on the surface of the electrode.

The term "precipitating" in accordance with the present invention relates to the process of forming a solid out of a solution, in particular it relates to the salting out of a soluble ionic form of the label from a solution, e.g. to form an insoluble salt precipitate. Typically, forming of an insoluble salt precipitate may involve the addition of counter ions to the ionic species in solution such that an insoluble salt may be formed that precipitates from the solution. The skilled artisan knows how to achieve precipitation of an ionic species from solution (Ting, 2009)

Specifically, according to the method of the invention, the precipitating in step iv) may comprise contacting a counter ion with the solubilized part of the label to allow for forming of an insoluble salt that precipitates from the solution. More specifically, this may involve adding a solution comprising a counter ion, e.g. a solution comprising an anionic species, particularly chloride, to the solubilized part of the label. The solution may comprise the counter ion in a suitable amount, e.g. in the range of 0.01 to 1 M, typically 0.1 M. Alternatively, the counter ion may be stored in a dry state and become solubilized in the fluid sample. Alternatively, the counter ion may be already available in the fluid sample.

More specifically, the insoluble salt precipitate of the label may be deposited on the surface of the electrode. More specifically, the insoluble salt precipitate and/or metal atoms of the label may also diffuse into layers of the electrode that are near to the surface of the electrode. Even more specifically, the insoluble salt precipitate of the label may include insoluble salts of silver or the like, e.g. silver chloride, silver (I) oxide, silver sulfide, silver bromide or silver iodide.

The term "insoluble salt" may refer to a salt exhibiting a solubility in water of less than 5 mg·l$^{-1}$ at 25 °C, preferably less than 2 mg·l$^{-1}$ at 25 °C, more preferably a solubility between 0.0001 and 2 mg·l$^{-1}$ at 25 °C.

The term "precipitating at least part of the label" refers to precipitating at least a measureable amount of the label.

The term "analyte complex" used according to the present invention refers to a complex comprising the analyte and the binding agent. The analyte complex may be formed on the surface of the electrode in particular on the surface that has been exposed to the fluid sample or is exposable to the fluid sample. The analyte complex may typically form upon binding of the analyte to the binding agent.

The detecting of the at least one analyte by electrochemical detection in step (v) typically comprises a pulse voltammetry (PV) measurement. More typically, the detecting comprises a differential pulse voltammetry (DPV) measurement, even more typically DPV with a forward scan, e.g. between -0.25 to 0.6 V or -0.25 to 0.25 V. Even more typically, the detecting involves monitoring oxidation of the metal to metal ions by PV, particularly by DPV. For example, the PV and/ or the DPV measurement may be performed versus an Ag/AgCl reference electrode. In particular, the PV and/or DPV measurements may be recorded within a range between -0.8 V to 1.0 V using a suitable pulse duration in the ms range, and/or a step between 5 and 15 mV, an amplitude between 10 to 200 mV and/or a scan rate of 5 to 50 mV s$^{-1}$. In particular using e.g. -0.25 V to 0.6 V with $t_{puls}$ = 50 ms, $E_{step}$ = 10 mV, $E_{amplitude}$ = 80 mV and/or scan rate = 20 mV·s$^{-1}$.

The method according to the invention further comprises a step of dissolving at least a part of the label by oxidation prior to precipitating at least a part of the label in step iv). "Dissolving" in particular refers to the transition of at least a part of the label from a solid form to a solubilized form, i.e. from a metal form to an ionic form. The solubilized form may dissolve in the fluid sample or any other suitable liquid surrounding or contacting the electrode surface, such as water, buffer, body fluids. By dissolving at least a part of the label it may be meant that an ionic species of the label, in particular a cationic species of the label, dissolves in the fluid sample or any other liquid surrounding or contacting the electrode surface.

The step of dissolving at least a part of the label by oxidation may specifically relate to obtaining a soluble form of the label, in particular an ionic form, more particularly a form that is soluble in the sample. For example, by using Ag nanoparticles as the label and by dissolving at least a part of the label by oxidation, $Ag^+$ cations may be obtained. The dissolving takes place electrochemically. In particular, the dissolving comprises applying a positive potential to the electrode, in particular the working electrode. In line with the method according to the invention a voltage, particularly a constant voltage, suitable to oxidize the metal nanoparticle is applied. According to the invention, a positive potential higher than the oxidation potential of the respective label, i.e. the metal nanoparticle, is applied. If surface-modified metal nanoparticles are used as labels, higher potentials than the oxidation potentials of the respective metal itself may be used. In particular, the positive potential may be in the range of 0.7 to 1.5 V, more particularly 1.0 to 1.5 V, more particularly 1.25 V. The positive potential may be applied for a suitable period of time, such as several 10 seconds to 120 seconds, e.g. 60 s. Typically, the applied voltage results in the formation of ions of the metal nanoparticle. Hence, the applied voltage results in the direct oxidation of the metal nanoparticle. In other words, the applied voltage may directly result in the formation of ions of the metal nanoparticle, in particular without requiring any additional substance. This means that the applied voltage typically leads to direct oxidation of the metal nanoparticle wherein a soluble ionic species of the metal nanoparticle is formed thereby dissolving at least part of the label. The term "direct oxidation" refers to oxidation of the metal nanoparticle caused by the applied voltage leading to an electron flow from the metal nanoparticle to the electrode. An electron flow from the metal nanoparticle to the electrode may refer to a transfer of electrons from the metal nanoparticle in the label to the electrode, in particular the working electrode, wherein the electrons generated by oxidizing the metal atoms in the nanoparticle to metal ions are transferred from the metal nanoparticle to the electrode, in particular the working electrode.

Direct oxidation may solely be caused by the applied voltage. Even more typically, there is no involvement of an additional substance, such as a redox mediator, or any further metal or ionic species in the oxidation of the metal nanoparticle and/or in the electron transfer from the metal nanoparticle to the electrode. Still even more typically, there is no redox mediator substance, for example gold atoms or gold ions, involved and/or there is no galvanic exchange between any mediator substance and the metal nanoparticle involved in the direct oxidation of the metal nanoparticle.

Dissolving at least a part of the label may be advantageous as the label may become more readily available for precipitating onto the surface of the electrode. The amount of label for precipitation may thereby be maximized. Maximizing the amount of label precipitated onto the surface of the electrode may enhance sensitivity of the detection.

Advantageously, dissolving the label used in the invention, in particular silver, is possible using less oxidative and toxic compounds as compared to gold. Moreover, electrochemical dissolution of the label may take place using a smaller potential and without requiring the addition of HCl.

The method of the invention may comprise applying a negative potential following the precipitating in step (iv), typically prior to step (v) comprising differential pulse voltammetry (DPV) measurement. In particular, the solubilized metal ions of the label may be reduced by applying a negative potential and metal atoms may be deposited on the surface of and/or diffuse into the electrode. More particularly, applying a negative potential refers to potential in the range of -0.5 V to -1.0 V, even more particularly a potential of -0.8 V, in order to achieve reduction of the metal ions. Still even more particularly, applying a negative potential is performed once, e.g. in a reduction single step. Advantageously, a single reduction step may be sufficient to achieve conversion of the solubilized metal ions to metal atoms on the surface of the electrode.

In the method according to the invention, the surface of the electrode exposable to the fluid sample may be coated, particularly coated with a linker as described herein above, more particularly streptavidin coated. Alternatively, the surface of the electrode exposable to the fluid sample may be uncoated. In case of a coated electrode surface, the coating may be applied by any suitable methods known to the person skilled in the art including. In the case of a streptavidin coating, the streptavidin can be added to the electrode material previous to fabrication of the electrode, e.g. before screen printing of the electrode. Alternatively, it can be adsorbed on the surface of the electrode. Typically, the electrode materials suitable for use in the present invention include carbon or modified carbon materials. More typically, the working electrode comprises a single electrically conductive material, such as carbon or modified carbon materials.

In addition, the surface of the electrode exposable to the fluid sample may further comprise a polypeptide, in particular a polypeptide engaging in non-specific interactions with the surface of the electrode and/or the coating, e.g. including BSA and the like. A coating of the surface of the electrode exposable to the fluid sample may be advantageous as it may serve as a linker to efficiently couple the binding agent to said surface of the electrode. The coating and/or said polypeptide may further serve to reduce unspecific interactions between the detection agent and/or components present in the sample with said electrode surface.

The term "surface of the electrode exposable to the fluid sample" according to the present invention may be understood as the side of the electrode surface that is contacted with the fluid sample and/or the analyte. In particular, the term "surface of the electrode exposable to the fluid sample" may relate to the surface of the working electrode configured to face or to be contacted with the sample.

The present application further discloses a working electrode of an analyte sensor, wherein the surface of the working electrode comprises at least a linker for coupling the binding agent. The working electrode is configured to allow for the formation of a detection complex comprising the detection agent, the binding agent and the analyte on the surface of the electrode exposable to a fluid sample. The detection agent comprises at least the further binding agent and a label, wherein the label comprises a metal nanoparticle with a standard redox potential $E°$ between 0 V and 1.2 V. The term "configured to allow for the formation of a detection complex" may refer to any kind of set up that enables contacting the detection agent, the binding agent and the analyte with the surface of the electrode exposable to the fluid sample upon exposure to or addition of the fluid sample. The binding agent may be immobilized on the surface of the electrode prior to the addition of the sample. The detection agent may be localized in close proximity to the surface of the electrode exposable to the fluid sample. In particular, the detection agent may be stored in a dry state and solubilized upon the addition of the fluid sample and/or by fluid the sample. More particularly the detection agent may be transported from its original location to said surface of the electrode upon the adding of the fluid sample and/or by the fluid sample. The possibility for dry storage of agents is advantageous as it allows for long-term storage and stocking and is therefore particularly suitable for use in point of care devices.

The term "working electrode" as used herein, may refer, without limitation, to the electrode of the analyte sensor that is sensitive for the analyte.

In particular, the working electrode in the present disclosure may be configured to allow for the formation of a detection complex also referred to as a sandwich complex comprising the detection agent and the analyte complex. The detection complex may be present or may form on the surface of the working electrode only and may typically be absent from any further electrodes or electrode surfaces, e.g. the surfaces of the counter electrode and/or the reference electrode may not comprise a detection complex.

A suitable reference electrode according to the present disclosure may be an Ag/AgCl electrode. Further, the working electrode may comprise a electrically conductive material in particular a carbon-based material; more particularly the working electrode may comprise a screen printed carbon electrode (SPCE). More particularly, the screen printed carbon electrode may be coated or uncoated. SPCEs are commercially available e.g. from Metrohm AG. Typically, the working electrode comprises a single electrically conductive material such as a carbon-based material, More typically, the working electrode does not comprise gold or a gold layer. The surface of the working electrode exposable to a fluid sample may be streptavidin coated. This coating may be advantageous as it may ensure efficient coupling of a binding agent comprising a biotin moiety.

Moreover, the present application discloses to an analyte sensor for detecting at least one analyte in a sample by electrochemical detection, comprising at least one working electrode as specified herein. The analyte sensor may further comprise at least one reference electrode. Typically, the reference electrode may be an Ag/AgCl reference electrode.

The present application also discloses a system comprising said analyte sensor. The system further comprises at least a controlling unit and at least an evaluating unit.

The present invention further relates to a device for detecting at least one analyte by electrochemical detection, comprising at least one working electrode as described elsewhere herein; and operatively linked thereto an analyzing unit configured for applying a voltage at the electrode suitable to oxidize the metal nanoparticle, and configured for measuring the detectable signal produced by the detection agent when detecting the at least one analyte; and optionally an evaluating unit. The term "device" as used herein typically relates to an apparatus or system that shall comprise at least the aforementioned means.

The term "operatively linked" as used herein thus, preferably, means functionally linked. Depending on the means to be used for the device or system of the present invention, said means may be functionally linked by connecting each mean with the other, e.g. by means which allow electrical potential or current transmission and/or data transport in between said means, e.g., by glass fiber cables, and/or by other cables for high throughput data transport and/or for electrical potential or current transmission. Nevertheless, wireless data transfer between the means is also envisaged by the present invention, e.g., via LAN (Wireless LAN, W-LAN).

As used herein, the term "analyzing unit" relates to a unit of a device that is configured to perform at least the functions indicated above, i.e. (i) applying a voltage at the electrode suitable to oxidize the metal nanoparticle, and (ii) measuring the detectable signal produced by the detection agent when detecting the at least one analyte. Typically, the analyzing unit of the invention may comprise or may be a potentiostat. More typically, the analyzing unit may be operatively linked to an evaluating unit or a data output device. Typical data output devices include a printer, a monitor or a display, specifically a display of a computer, of a mobile device, or a tablet.

As used herein, the term "evaluating unit" typically relates to a unit of a device comprising a database and an implemented computer program evaluating that data. More typically, the evaluating unit is adapted to determine the amount of the analyte by comparing the detectable signal measured by the analyzing unit to a reference. Thus, the evaluating unit specifically is adapted to determine the concentration or amount of the at least one analyte by using an evaluation algorithm and/or to store reference values and or reference curves and/or reference areas. More typically, the evaluating unit is adapted to perform all calculations and evaluations required to release a value of a concentration or the value of an amount of an analyte in a fluid sample; even more typically, the evaluating unit comprises a data output device as specified herein above. Still even more typically, the evaluating unit comprises at least one data processing device, specifically at least one computer, microcomputer, mobile device or tablet.

The term "reference" relates to a value or values of known features of the at least one analyte. In particular, the known features may relate to a detectable signal produced by the detection agent in a sample comprising a known amount of the at least one analyte, also referred to as reference amount. The reference may be a threshold amount. The amount of an analyte in a fluid sample may be higher or lower than the threshold amount. A reference amount may, typically, be derived from a reference sample or a group thereof. The reference may be a calculated reference, typically the average or median, for the relative or absolute amount of the at least one analyte in the fluid sample. The absolute or relative amounts of the at least one analyte can be determined as specified elsewhere herein, specifically by electrochemical detection. How to calculate a suitable reference value, specifically, how to calculate the average or the median of a reference sample or a group of reference samples, is well known in the art. The term "reference" may relate to a single reference values, several reference values, a reference curve and /or a reference area. According to the present invention, the reference may in particular relate to a peak area of a reference sample or to a peak height of a reference sample, more particularly to the peak area of a reference sample. The peak may in particular relate to a peak of a value determined by voltammetry, specifically to a peak current determined by differential pulse voltammetry.

Typical systems or apparatuses according to the invention include: a point of care device, a fluidic system such as a microfluidic system, a lab-on-a chip, and the like, in particular a microfluidic system. Microfluidic devices and systems are known in the art. Typically, a microfluidic system comprises a microfluidic device, and one or more pumping device(s). In particular, a microfluidic device includes at least one flow cell, at least one internal flow channel, and at least one port in fluid communication with the flow channel. More particularly, the microfluidic device comprises at least a microfluidic flow cell. Suitable microfluidic flow cells include thin layer flow cell SPCEs such as for example DRP-TLFCL110 and FLWCL available from DropSens (DS). Suitable microfluidic devices and systems are known in the art and disclosed for example in EP 2016997 A1 or EP 3610947A1. WO 2017/216339 A1 discloses an alternative suitable test system comprising a fluidic device for analyzing liquid samples i.e. body fluids.

In accordance with the present invention is the use of a metal nanoparticle with a standard redox potential E° between 0 V and 1.2 V as a label in electrochemical detection of an analyte comprising PV or DPV measurements as defined by the claims.

Further characteristics, details and advantages of the invention follow from the wording of the claims and from the following description of practical examples on the basis of the drawings.

Figures

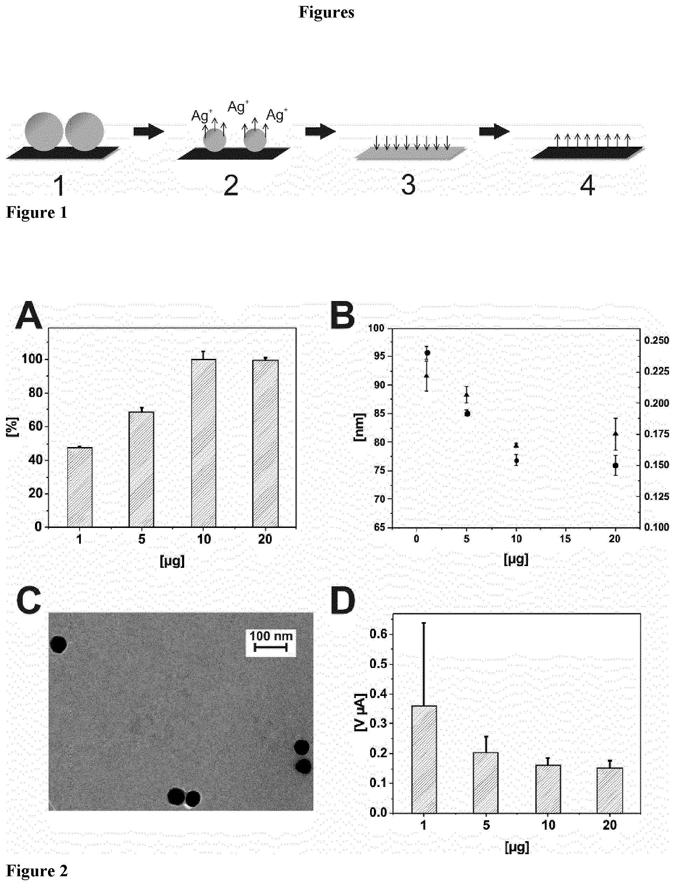

Figure 1 shows a schematic representation of electrochemical detection using AgNPs. The detection of AgNPs consists of four steps: In the first step, the AgNPs used as labels are located close to the WE surface. In a second step, the silver nanoparticles are oxidized in presence of KCl and AgCl precipitates immediately on the surface. In step three, the Ag⁺ ions are reduced upon formation of a silver layer. The oxidation in the last step is monitored by DPV.

Figure 2 depicts a plot of the ratio of maximum absorbance in presence of 2 M NaCl to the maximum absorbance of the pure AgNP solution against the amount of probe AB used in the modification process (A). Non-blocked AB-AgNPs were used for this measurement. Error bars represent mean values +1σ and were calculated based on three parallel measurements (n = 3). Change of hydrodynamic diameter (circle, left axis) and PdI (triangle, right axis) of blocked AB-AgNPs with changing amount of probe AB (B). Error bars represent mean values ± 1σ and were calculated based on three parallel measurements (n = 3). Exemplary TEM image of the blocked AB-AgNP modified with 10 μg probe AB, scale bar represents 100 nm (C). Plot of peak area of the bioassay using a constant AG concentration of 100 ng·mL⁻¹ against the amount of probe AB used in the modification process of AgNPs (D). Error bars represent mean values + 1σ and were calculated based on three parallel measurements on three different SPCEs (n = 3).

Figure 3. depicts the change of hydrodynamic diameter (circle, left axis) and PdI (triangle, right axis) of AB-AgNPs (modified with 10 μg AB, blocked with BSA) over 56 days after modification (left plot). Stability of the electrochemical signal of the bioassay using a constant AG concentration of 100 ng·mL⁻¹ over 56 days.

Peak area was normalized to the signal right after the modifictaion (right plot). Error bars represent mean values $\pm 1\sigma$ and were calculated based on three parallel measurements on three different SPCEs (n = 3).

Figure 4    depicts depicts exemplary differential pulse voltammograms of the bioassay with AgNPs for differently concentrated antigen solutions (0 - 1000 ng·mL$^{-1}$, light to dark) normalized with respect to the baseline (left) and plot of corresponding peak area against logarithm of antigen concentration with logistic fit (solid line) and corresponding parameters (right). In the left plot, the x-axis depicts the applied potential [V], the y-axis the normalized current [μA], in the right plot the x-axis represents the antigen concentration [ng mL$^{-1}$], the y-axis represents the peak areas [V μA]. The intersection of the dashed line with the measuring curve indicates the LOD. Standard deviations were calculated based on five parallel measurements on five different SPCEs, while outliers were removed after Q-test (confidence interval 95%). Error bars represent mean values $\pm 1\sigma$ (n $\geq$ 4).

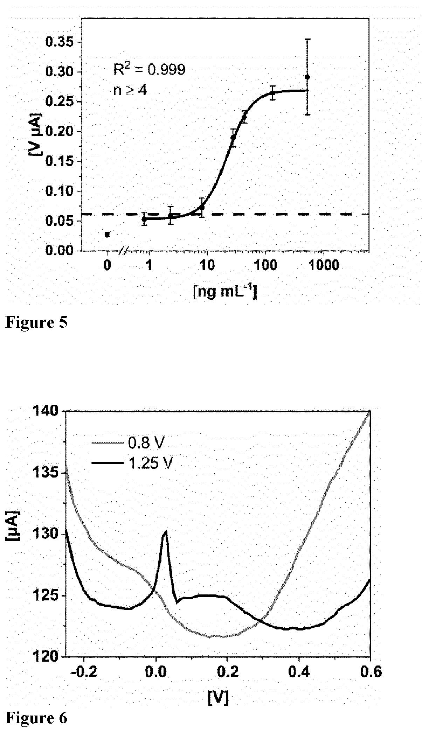

Figure 5    shows a plot of peak area ([V μA], y-axis) against logarithm of antigen concentration ( [ng mL$^{-1}$], x-axis) in spiked human serum samples with logistic fit (solid line) and corresponding parameters. The intersection of the dashed line with the measuring curve indicates the LOD. Standard deviations were calculated based on five parallel measurements on five different SPCEs, while outliers were removed after Q-test (confidence interval 95%). Error bars represent mean values $\pm 1\sigma$ (n $\geq$ 4 5).

Figure 6    shows the DPV of the performance of the Bioassay with 1000 ng·mL$^{-1}$ analyte concentration with oxidation at 1.25 V for 30 s (black) versus oxidation at 0.8 V for 30 s (grey); the x-axis depicts the applied potential [V], the y-axis the current [μA].

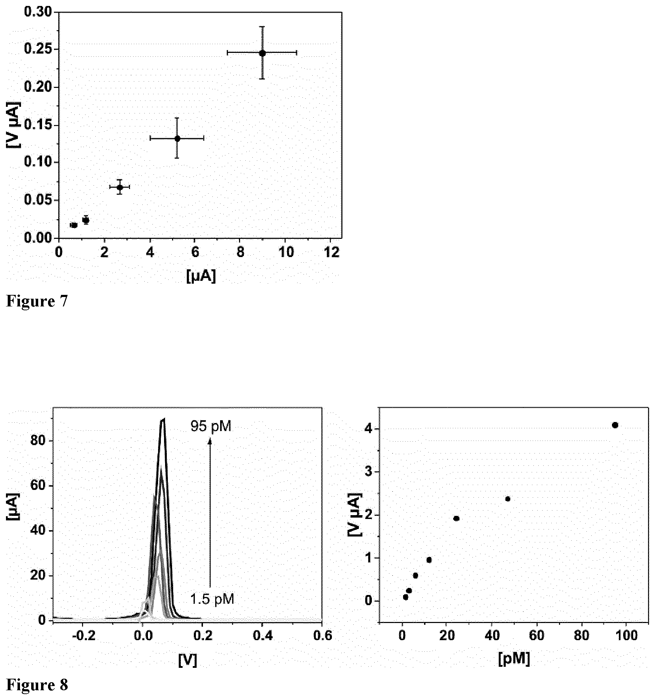

Figure 7    shows the correlation of peak area ([V μA], y-axis) and peak height ([μA], x-axis). Standard deviations of peak area and peak height were calculated based on five parallel measurements on five different SPCEs, while outliers were removed after Q-test (confidence interval 90%). Error bars represent mean values $\pm 1\sigma$ (n = 5). Each data point correlates to one AG concentration.

Figure 8    shows DPV measurements ([μA], y-axis) of various AgNP solutions ([pM], x-axis), dried on top of DropSens electrodes (left) and plot of peak area with AgNP concentration (right).

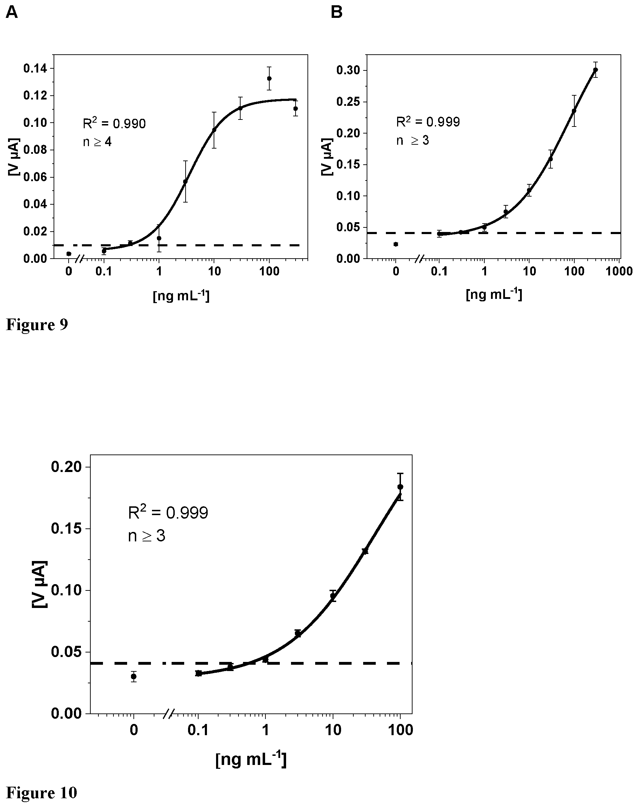

Figure 9    shows the dose-response curve for the flow chips generation one with pre-incubation of the AG and AB-AgNP (A) and the flow chips with dried AB-AgNPs in trehalose matrix (B). The plot of peak area against logarithm of antigen concentration in PBS buffer (50 mM phosphate) with logistic fit (solid line) and corresponding parameters is depicted. The intersection of the dashed line with the measuring curve indicates the LOD. Standard deviations were calculated based on parallel measurements using different flow chips (A: flow chip generation one with pre-incubation of AG and AB-AgNP, B: flow chip generation two with dried AB-AgNP in trehalose matrix), while outliers were removed after Q-test (confidence interval 95%). Error bars represent mean values $\pm 1\sigma$.

Figure 10    depicts generation two measurements (with dried AB-AgNPs) in human serum samples, spiked with the respective AG concentration. Shown is the plot of peak area against logarithm of antigen concentration in human serum with logistic fit (solid line) and corresponding parameters. The intersection of the dashed line with the measuring curve indicates the LOD. Standard deviations were calculated based on parallel measurements using different flow chips (generation two with dried AB-AgNP in trehalose matrix), while outliers were removed after Q-test (confidence interval 95%). Error bars represent mean values $\pm 1\sigma$.

## Examples

The following examples serve to illustrate the invention.

Example 1: Chemicals and Instruments

The biotinylated capture antibody (polyclonal proBNP sheep-IgG-biotin), antigen (NT-proBNP (1-76) amid) in buffer or human serum, probe antibody (monoclonal NTproBNP mouse-IgG) and antibody modified gold nanoparticles (monoclonal NTproBNP mouse-IgG-gold) were provided by Roche Diagnostics GmbH, Mannheim, Germany. Citrate capped silver nanospheres (d = 50 nm, 0.022 mg·mL$^{-1}$) were purchased from nanoComposix (www.nanocomposix.com). Hydrochloric acid (HCl, 0.1 M, 1 M), sodium chloride (NaCl, p.a), disodium hydrogen phosphate (Na$_2$HPO$_4$ 2 H$_2$O, p.a.)

and potassium dihydrogen phosphate ($KH_2PO_4$, p.a.) were ordered from Merck (www.merckmillipore.com). Bovine serum albumin (BSA, >96%) and Tween 20 (>97%) were sup-plied from Sigma Aldrich (www.sig-maaldrich.com). Potassium chloride (KCl, p.a.) was obtained from Roth (www.carlroth.com). 4-(2-hydroxyethyl)-1-piperazineethanesulfonic acid (HEPES, ≥99%) was acquired from VWR (de.vwr.com) and sodium hydroxide (NaOH, 1 M) was bought from Labochem international (www.labochem.de). HEPES buffer consisted of 10 mM HEPES and was ad-justed to a pH of 7.4. HEPES blocking buffer was prepared by addition of 0.1% (w/v) BSA to this HEPES buff-er. Phosphate buffered saline (PBS) consisted of 137 mM NaCl, 2.7 mM KCl, 38 mM Na2HPO4·2 H2O and 12 mM KH2PO$_4$ with a pH of 7.4. For PBST washing buffer 0.05% (w/v) Tween 20 were added to this PBS.

All electrochemical measurements were performed using screen-printed carbon electrodes (SPCE) bare (DRP-110) or with streptavidin coated working electrode (DRP-110STR, both: Metrohm AG, www.dropsens.com) and an EmStat blue potentiostat with corresponding software (PalmSens, www.palmsens.com). For nanoparticle modification, the TermoMixer comfort (Eppendorf, online-shop.eppendorf.de) was used. A Plate reader Synergy Neo2 Hybrid Multi-Mode Reader from BioTek Instruments (BioTek Instruments Inc., www.biotek.de), a Malvern Zetasizer Nano-ZS (www.malvern.com) and a 120 kV Philips CM12 (www.fei.com) transmission electron microscope were employed for characterization of the modified AgNPs.

Example 2: Electrochemical Detection of Gold and Silver Nanoparticles

To test the electrochemical detection of both metallic nanoparticles (gold and silver), 10 $\mu$L of the respective NP solution (diluted in HEPES buffer) are dried on top of the working electrode of the SPCE (DRP-110). Immediately afterwards, 50 $\mu$L of 0.1 M HCl for the gold measurement or 0.1 M KCl for silver respectively, are added and the electrochemical measurement is started. The measurement procedure for gold nanoparticles consists of a pretreatment and the actual differential pulse voltammetry. First a voltage of 1.25 V is applied for 60 s, then DPV is performed from 1.25 V to 0 V with $t_{puls}$ = 50 ms, $E_{step}$ = 10 mV, $E_{pulse}$ = 80 mV and scan rate = 20 mV·s$^{-1}$. For silver nanoparticles an equivalent, but slightly different procedure is used. For pretreatment, a voltage of 1.25 V is applied for 60 s, then -0.8 V for 30 s. The DPV is recorded from -0.25 V to 0.6 V with same settings (Hao et al., 2011).

Example 3: Modification of Silver Nanoparticles

For the AgNP modification, a modified procedure of Szymanski et al. (2013) was utilized. A volume of 1 mL of AgNP solution (0.02 mg·mL$^{-1}$) is centrifuged for 10 min at 10,000 g. The supernatant is discarded and the pellet resuspended in 1 mL HEPES with different amount of probe AB. After incubation at room temperature with gentle mixing (350 rpm) for 2 h in the dark, the nanoparticles are centrifuged once again for 10 min at 10,000 g. The supernatant is discarded and the pellet resuspended in 1 mL HEPES or HEPES blocking buffer. For characterization of those particles, UV/Vis measurements are performed first. Four AgNP solutions, modified with 1, 5, 10 and 20 $\mu$g ABs in HEPES are adjusted to the same concentration (according to maximum absorbance). Then, 200 $\mu$L of each solution are pipetted into a transparent 96 well MTP (Greiner, shop.gbo.com) and the maximum absorbance at 340 nm is measured. After addition of 50 $\mu$L 2 M NaCl and mixing for 2 min, the maximum absorbance is measured once again. Four additional AgNP solutions are modified with 1, 5, 10 and 20 $\mu$g ABs in HEPES blocking buffer. These blocked AB-AgNPs are used for all further experiments. The characterization is completed by DLS measurements at 25 °C in disposable PMMA cuvettes (semi-micro), TEM measurements and the performance of the bioassay with 100 ng·mL$^{-1}$ AG concentration.

Example 4: Performance of the Bioassay

Prior to usage, the SPCEs (DRP-110STR) are washed three times with 50 $\mu$L PBS buffer. For the bioassay, 10 $\mu$L of the respective solution are dropped onto the working electrode. After incubation for 1 h at room temperature under water saturated atmosphere the solution is removed and the electrode washed three times with 50 $\mu$L PBST. After drying with nitrogen, the next solution is added. The exact sequence of solutions is shown in Table 1. After step 4, the electrodes are washed additionally with PBS and double distilled water. Directly prior to the electrochemical measurement, the electrode is dried with nitrogen and the three-electrode area covered with 50 $\mu$L of a 0.1 M HCl or KCl, respectively. The measurement is performed as described above.

**Table 1:** Sequence of steps with respective solutions, all diluted in PBS.

| Step | Solution[a] |
|---|---|
| 1 immobilization of capture antibody | 25 $\mu$g·mL$^{-1}$ biotinylated capture AB |
| 2 blocking | 1% (w/v) BSA |
| 3 antigen binding | 0-3000 ng·mL$^{-1}$ AG |

(continued)

| Step | Solution[a] |
|---|---|
| 4 Probe antibody binding | 7.1 ng·mL$^{-1}$ AuNP-tagged probeAB |
| | 20 μg·mL$^{-1}$ probe AB modified AgNP[b] |

[a] First three solutions were diluted in PBS, labelled probe ABs were diluted in HEPES.

[b] Due to different concentration data given by the manufacturer, dilutions of AB-AuNP and AB-AgNP cannot be given in a consistent form. However, this dissimilar approach did not interfere with assay optimizations.

Example 5: Electrochemical Detection of Metallic Nanoparticles

For the electrochemical detection of both kinds of metallic nanoparticles, already published procedures were adjusted. The processes on the electrode surface are shown in Fig. 1. The gold nanoparticle detection was discussed in more detail by La Escosura-Muñiz et al., 2011. In the first step, the AuNPs are dried on the working electrode of the SPCE. After addition of hydrochloric acid, the particles dissolve upon oxidation at 1.25 V (step 2). Hereby, a gold chlorido complex forms near the electrode surface. In the third step, the reduction of bound Au$^{3+}$ ions at 0.25 V is monitored by DPV. The hydrochloric acid is inevitable for the initial oxidation of the very stable AuNPs, because the complex is only formed at a pH around one.

For the silver nanoparticle detection (Fig. 1) an equivalent but slightly more complex approach was used (Hao et al., 2011). Here, the AgNPs dissolve by oxidation at 1.25 V (step 2) after drying (step 1). Due to the higher instability of silver compared to gold, already 0.8 V would be enough to oxidize the bare nanoparticles. However, the presence of proteins on the NP surface requires a higher potential to clean the NP surface and thus get a measureable DPV signal. The Ag$^+$ ions then form an AgCl precipitate on the electrode surface with the present chloride, which hinders the diffusion away from the electrode surface (Ting et al., 2009). The third step consists of a reduction at -0.8 V to form a silver layer, which penetrates into the pores of the electrode material. In the last step, the oxidation around 0.025 V is monitored by DPV. This additional step renders the electrochemical detection considerably more sensitive and sharper peaks are obtained. The main advantage of silver over gold is that no addition of hydrochloric acid is needed due to its higher chemical instability. Since chloride ions are contained in biological samples, this enables an one-step analysis.

As proof of concept, differential pulse voltammetry was performed with both different mNPs. A concentration dependence of peak area and height was found (Fig. 7, Fig. 4 and 8: left side). Due to marginal smaller errors, the peak area was used for all following data evaluation. The plot of peak are against NP concentration shows the same course for both metals and is shown exemplary for AgNPs in Fig. 8. First, the signal increases linearly with the amount of NP on the surface, while a constant value is reached after saturation of the electrode surface. This shows that the DPV detection method can be used for both, gold and silver nanoparticles.

Example 6: Performance of the Bioassay with Gold Nanoparticles

First, different techniques to immobilize the AG on the working electrode were tested. No gold signal at 0.25 V was obtained neither for a direct adsorption of the antigen (AG), nor for a covalent binding of the capture AB using EDC/NHS chemistry. Adsorption processes are highly dependent on the protein-electrode combination, which is used. In this case, the AG was washed away even after incubation overnight, since the interaction was too weak. The covalent AB immobilization itself worked according to impedance studies. The absence of a gold signal could be due to the blocking of the electrode by the pyrene butyric acid, which was used as anchor moiety, and the increased distance between NP label and electrode surface. Thus, a third approach exploiting streptavidin/biotin binding on purchased streptavidin-functionalized electrodes (DRP-110STR) was performed. The streptavidin is not coated on top of the electrode, but rather included in the conductive material. With this method, the AG was bound to the electrode and the presence of gold nanoparticles was measured at 0.25 V due to decreased NP-electrode distance and blocking of the electrode. In the next step, the capture AB concentration was varied and an optimal concentration of 25 μg·mL$^{-1}$ was found. The working electrode seems to be completely covered and further increasing the capture AB concentration did not change the signal. Moreover, different dilutions of the purchased AuNP-tagged probe AB solutions were used for the bioassay. Due to a drastically higher signal, the probe ABs were used in a 1:10 dilution. Using the AuNP solution without any dilution worsened the signal-to-noise (S/N) ratio due to an increase in background. With these optimized parameters, the bioassay was performed.

For better visualization of the concentration dependent peak heights, the voltammograms are normalized with respect to the baseline, which means the current difference to the current measured at 0.45 V is plotted. In the plot of the peak area against logarithm of AG concentration, a sigmoidal binding curve can be seen . The limit of detection (LOD) was calculated using the logistic fit parameter for the lower border A1 and the standard deviation of the blind SD(blind):

$$LOD = A1 + 3 \cdot SD(blind) \qquad\qquad (1)$$

A concentration value of 26 ng·mL$^{-1}$ was calculated based on the logistic fit. The mean error of all measurements is 17% and the dynamic range extends over nearly two orders of magnitude from 25 to 1000 ng·mL$^{-1}$. Overall, the gold bioassay is easy to perform and provides reliable results. However, the addition of hydrochloric acid is cumbersome considering a true POC application and the detection is not sensitive enough to use it for blood analysis, where NT-proBNP is contained in the pg·mL$^{-1}$ range. To solve both problems the metal used was changed to silver.

Example 7: Modification of Silver Nanoparticles

Purchased silver nanoparticles were modified with different amounts of probe AB to find the optimal loading density. UV/Vis analysis was performed to prove the passive adsorption of AB to AgNPs worked and see the stabilizing effect of the proteins (Hao et al., 2011).

In high ionic strength media, bare NPs tend to aggregate and in consequence, their color changes from yellow to transparent. For the different modifications (with 1 to 20 $\mu$g·mL$^{-1}$ AB), the maximum absorbance after addition of 2 M NaCl was divided by the original maximum value. Non-blocked particles had to be used for this study, since AB-AgNPs would not show any signal change after blocking. The ratio of both maxima, shown in Fig. 2 (A), reaches one when 10 $\mu$g AB are used in the modification and stays constant for higher amounts. This means, that a minimum modification concentration of 10 $\mu$g·mL$^{-1}$ is needed to preserve the NPs from agglomeration, i.e. to cover the NP surface completely. In the DLS measurements Fig. 2 (B) it can be seen that the hydrodynamic diameter and Polydispersity Index (PdI) decrease until a constant value of around 75 nm and 0.160 is reached for AB-AgNPs modified with 10 $\mu$g. A complete coverage of the surface with ABs decreases the overall size and increases uniformity, because less BSA adheres to the nanoparticle during blocking. The hydrodynamic diameter increases by about 25 nm compared to the bare NPs (52 nm, given by the manufacturer) due to protein uptake.

The TEM images Fig. 2 (C) show that the AgNP core size is not affected by the modification procedure and no aggregates are formed. The differently modified AB-AgNPs were then used in the bioassay with a constant AG concentration of 100 ng·mL$^{-1}$ Fig. 2 (D). With increasing amount of probe AB, the peak area decreases slightly, but the standard deviations show a huge improvement. This proposes an increased uniformity of the NPs with increasing surface coverage. In the following, AgNPs modified with 10 $\mu$g probe AB were used, since their surface is completely covered and they show excellent uniformity. Since silver nanoparticles are known to be quite unstable due to aggregation and oxidation, a stability study was performed next. Dynamic light scattering (DLS) measurements and the bioassay with a constant AG concentration were performed over eight weeks after modification (Fig. 3).

The hydrodynamic diameter and PdI decrease minimally within the first three days (Fig. 3 left). Afterwards, a constant value is reached. This is likely caused by a slow release of BSA loosely attached to the particle in the first days after modification until an equilibrium is reached. Application of the prepared and stored AB-AgNPs in the bioassay (Fig. 3 right) shows that they are stable as signals do not change within the margin of error observed for a period of 4 weeks. After eight weeks, the peak area drops to 50% of the original value. This indicates that the overall assay and signal enhancement strategy is rugged, but for final application, further studies are needed to increase the storage stability of the particles. The short-term stability of non-blocked AB-AgNPs were also tested. For short-term stability, blocking with BSA made no significant difference. However, to optimize long-term stability and avoid any unspecific binding in the final application, the blocked AB-AgNPs were used for all further measurements.

Example 8: Performance of the Bioassay with Silver Nanoparticles

The bioassay was performed with silver nanoparticle-labelled probe AB, while using the optimum parameters found with the gold assay. Exemplary differential pulse voltammograms in an AG concentration range from 0 to 1000 ng·mL$^{-1}$ are shown in Fig.4 (left plot). These voltammograms are normalized with respect to the baseline using the current value at 0.05 V. The peaks have a considerably smaller full-width at half maximum (FWHM) compared to gold, which is due to the additional step in the detection. Moreover, the signal of the electrochemically more active silver arises at a ten-time smaller potential. This is always beneficial considering interferences in biological samples.

The plot of corresponding peak area against logarithm of AG concentration shows a sigmoidal curve (Fig. 4 right). Using the logistic fit and equation 1, a LOD of 4.0 ng·mL$^{-1}$ was calculated, which is around six-times lower than for the AuNP assay, while the reproducibility with a mean error of all measurements of 17% is comparable. Interestingly, the dynamic range between 4 and 100 ng·mL$^{-1}$ is narrowed. Considering the better analytical performance of the AgNP assay accompanied with the increased simplicity as no further solutions is needed, the AgNP bioassay highly valuable for a POC application.

Finally, demonstrating its true applicability, the AgNP bioassay was tested with real samples, specifically analyses were performed in human serum samples, spiked with different amount of NT-proBNP (Fig. 5). Most of the parameters are similar to those of the silver bioassay in buffer: the curve shape and with it, the dynamic range. The calculated LOD of 4.7 ng·mL$^{-1}$ is marginally higher due to background adsorption of serum proteins. The mean error of 15% is even slightly better. Thus, overall, the assay conditions and washing are suitable for application in real samples.

Two bioassays with metal nanoparticles for signal enhancement were investigated with respect to their applicability towards point-of-care sensing. In the case of AuNPs, these excel due to an excellent analyte concentration range, *i.e.* from 25 to 1000 ng·mL$^{-1}$ with very good signal to noise ratios. Moreover, it is well known that gold nanoparticles are easy to modify and stable over a longer period of time. However, due to their greater electrochemical activity, AgNPs provide a six-times more sensitive assay. Most importantly, the AgNP bioassay is significantly simpler and hence more adaptable to a POC setting as no further addition of any solution is necessary once it is used in a biological sample. Of importance here is also the long-term storage stability of the antibody-modified AgNPs.

In Figure 6, DPV measurements demonstrate the performance of the Bioassay. The Bioassay was conducted using 1000 ng·mL$^{-1}$ analyte concentration with oxidation at 1.25 V for 30 s (black) versus oxidation at 0.8 V for 30 s (grey); the x-axis depicts the applied potential [V], the y-axis the current [$\mu$A].

Example 9: Microfluidic System

In here, we report the successful transfer of the AgNP assay into a microfluidic system. After optimization of the experimental setup and conduct and adjustment of the assay parameters, a very low LOD of 0.27 ng/mL with a mean error of 25% (n = 5) was obtained (Fig. 9). This is an improvement by a factor of 15 in comparison to the assay performed on free electrodes (LOD of 4 ng/mL). The reproducibility may be improved but is a good representation of the many manually performed steps. The transfer of the original assay into a flow format bears significant implications on a future POCT as it demonstrates that reactions can all occur in flow and that the current AgNP signal generation system lends itself well for the envisioned future format. Most importantly, this flow system provides an LOD in a clinical relevant concentration range.

To obtain a flow cell an oval shape is cut out of double-sided adhesive tape (d = 100 $\mu$m) by a laser cutter. After gluing it together with a PMMA top, a rectangular shape is cut out. These channel tops are stored for later usage. Next, the streptavidin coated DropSens electrode (DRP-110STR) was washed three times with 50 $\mu$L PBS and 10 $\mu$L of capture AB (polyclonal proBNP sheep-IgG-biotin) were added to the working electrode and incubated for 1 h at room temperature. After washing three times with 50 $\mu$L PBST the electrode was dried under nitrogen and glued together with the channel top to form the finished flow cell.

Two pumps and a T-piece allow an exchange of solution without getting bubbles into the closed system. The flow chip is placed in a chip holder with in- and outlet. The outlet tubing leads into a waste or a buffer reservoir depending on the mode the system is operated in. The two different modes are called injection and withdrawal mode and depend on the pumping direction. While washing buffer and KCl are injected via the syringes, blocking buffer and AG/AB-AgNPs are injected from the right side in withdrawal mode in order to avoid bubbles and ensure small solution consumption. The AG + AB-AgNP mix consists_of 5 $\mu$L NT-proBNP (1-76) amid and 5 $\mu$L monoclonal NTproBNP mouse-IgG-silver,_incubated for 2 h at room temperature before injection. After filling of the microfluidic channel with KCl, the flow chip is taken out of the chip holder, connected to a potentiostat and the electrochemical measurement is performed. This last step was done since the current chip holder does not allow connection to the potentiostat.

During assay development, the general microfluidic setup and flow scheme, as well as flow velocity, oxidation potential and pre-incubation time were varied. The optimized parameters were used to establish a dose-response curve with different AG concentrations (Fig. 9) with respect to further use of this microfluidic system with probe AB dried directly in the channel(generation two); the final injected concentration of AG is given on the x-axis.

Figure 9 shows the dose-response curve for the flow chips generation one with pre-incubation of the AG and AB-AgNP (A) and the flow chips with dried AB-AgNPs in trehalose matrix (B; generation two).

For the flow chips generation one with pre-incubation of the AG and AB-AgNP (A) the microfluidic assay has a LOD of 0.27 ng/mL, which is a huge improvement compared to the free assay with 4.0 ng/mL. This sensitivity enhancement by a factor of 15 has two reasons. The AG/AB interaction is not limited by slow diffusion of the big nanoparticles due to small channel height, and the blind signal and standard deviation is decreased due to better and more intense washing. The mean error is 25%. There are two main weak points: due to an irregular baseline, peak picking is difficult and inconsistent pumping makes injection of constant volumes impossible. Still this is a proof of principle that the silver nanoparticle assay can be performed in one-step in flow and reaches a clinical relevant concentration range. As a side note, the signal intensity is lowered by around 50% compared to the assay on free electrodes, yet the signal-to-noise ratio is even better. This may be due to a reduction of working electrode area and change of oxidation potential during assay optimizations. Latter was necessary to inhibit bubble evolution in the channel.

For the flow chips with dried AB-AgNPs in trehalose matrix (Fig. 9 B; generation two), The calculated LOD is with 0.24 ng·mL$^{-1}$ very close to the 0.27 ng·mL$^{-1}$ for the microfluidic assay with pre-incubation of AG and AB-AgNP. Moreover, after optimizations of the procedure and flow chips, the relative error was decreased from 25% (n$\geq$4) to 9% (n$\geq$3) and the

logistic fit is considerably better ($R^2$ = 0.999).

The higher ground signal may probably be caused by residuals of dried NPs in the channel. The overall higher signal and clearer peaks enable a more reproducible data evaluation. This, in combination with the easier experimental conduct, leads to an excellent reproducibility.

The generation two measurements (with dried AB-AgNPs) were repeated in human serum samples, spiked with the respective AG concentration (Fig. 10). The LOD of 0.57 ng·mL$^{-1}$ is slightly higher than for the assay in buffer presumably because of higher errors of the blind due to protein adsorption. The mean standard deviation is excellent, merely 6% (n≥3).

**Cited literature**

Hao N, Li H, long Y, Zhang L, Zhao X, Xu D, Chen H-Y (2011) An electrochemical im-munosensing method based on silver nanoparticles. Journal of Electroanalytical Chemistry 656(1-2):50-54.

Khristunova Y, et al.; (2019) Preparation and Investigation of Silver Nanoparticle-Antibody Bioconjugates for Electrochemical Immunoassay of Tick-Borne Encephalitis. SENSORS, vol. 19, no. 9, page 2103.

La Escosura-Muñiz A de, Parolo C, Maran F, Mekoçi A (2011) Size-dependent direct electrochemical detection of gold nanoparticles: application in magnetoimmunoassays. Nanoscale 3(8):3350-3356.

Pollok NE, Rabin C, Walgama CT, Smith L, Richards I, Crooks RM (2020) Electrochemical Detection of NT-proBNP Using a Metalloimmunoassay on a Paper Electrode Platform. ACS Sens. 5: 853-860.

Pumera M, Aldavert M, Mills C, Merkoçi A, Alegret S (2005) Direct voltammetric determination of gold nanoparticles using graphite-epoxy composite electrode. Electrochimica Acta 50(18):3702-3707.

Szymanski M, Turner AF, Porter R (2010) Electrochemical Dissolution of Silver Nanoparticles and Its Application in Metalloimmunoassay. Electroanalysis 22(2):191-198.

Szymanski MS, Porter RA (2013) Preparation and quality control of silver nanoparticle-antibody conjugate for use in electrochemical immunoassays. Journal of immunological methods 387(1-2):262-269.

Ting BP, Zhang J, Gao Z, Ying JY (2009) A DNA biosensor based on the detection of dox-orubicin-conjugated Ag nanoparticle labels using solid-state voltammetry. Biosensors & bi-oelectronics 25(2):282-287.

**Claims**

1. A method for detecting at least one analyte by electrochemical detection, comprising:

   (i) contacting a fluid sample suspected to comprise the at least one analyte with the surface of an electrode comprising at least one binding agent capable of binding to the analyte;
   (ii) contacting the fluid sample suspected to comprise the at least one analyte with a least one detection agent, wherein the at least one detection agent comprises at least a further binding agent capable of binding to the analyte and a label, wherein the label comprises a metal nanoparticle with a standard redox potential E° between 0 V and 1.2 V;
   (iii) forming a detection complex on the surface of the electrode comprising at least the at least one binding agent, the at least one detection agent and the analyte;
   (iv) precipitating at least a part of the label onto the electrode surface; and
   (v) detecting the at least one analyte by electrochemical detection;
   wherein the method comprises a step of dissolving at least a part of the label by oxidation prior to precipitating at least a part of the label in step (iv), and
   wherein the step of dissolving at least a part of the label by oxidation comprises applying a voltage at the electrode suitable to oxidize the metal nanoparticle, wherein a positive potential higher than the oxidation potential of the respective metal nanoparticle is applied as this voltage, wherein the applied voltage results in direct oxidation of the metal nanoparticle.

**2.** The method according to claim 1, further comprising:
a step of forming an analyte complex on the surface of the electrode comprising the at least one binding agent and the analyte, prior or simultaneously to step (iii) of forming of the detection complex.

**3.** The method according to claim 1 or 2, wherein the label comprises or consists of a metal nanoparticle of silver or alloys thereof; specifically a silver nanoparticle, or a metal nanoparticle comprising or consisting of an silver/gold alloy; more specifically a citrate-capped or citrate-stabilized silver nanoparticle.

**4.** The method of claim 3, wherein the positive potential is applied as a voltage in the range of 1.0 to 1.5 V.

**5.** The method according to any of the preceding claims, wherein the at least one binding agent is immobilized on the surface of the electrode exposable to the fluid sample, preferably via a linker; preferably, wherein the at least one binding agent and/or the at least one further binding agent are selected from antibodies and fragments thereof, nucleic acids, aptamers, peptide nucleic acids (PNAs), receptor or ligand proteins or peptides, and enzymes; more preferably the binding agent and/or the further binding agent comprises an antibody, even more preferably an IgG.

**6.** The method according to any of the preceding claims, wherein the detection agent is stored in a dry state and solubilized upon the addition of the fluid sample.

**7.** The method according to any of the preceding claims, wherein the electrochemical detection in step (v) comprises pulse voltammetry measurements, in particular differential pulse voltammetry (DPV) measurement.

**8.** The method according to any of the preceding claims, wherein the method comprises applying a negative potential following precipitating in step (iv).

**9.** The method according to any of the preceding claims 1 to 8, wherein the precipitating in step (iv) comprises contacting a counter ion with the dissolved part of the label to allow for forming of an insoluble salt that precipitates from the solution.

**10.** A device for detecting at least one analyte by electrochemical detection, comprising:

a) at least one working electrode, wherein the surface of the working electrode comprises at least a linker for coupling the at least one binding agent, and wherein the working electrode is configured to allow for the formation of a detection complex comprising at least one detection agent, the at least one binding agent and the analyte on the surface of the electrode exposable to a fluid sample, wherein the at least one detection agent comprises at least one further binding agent and a label, wherein the label comprises a metal nanoparticle with a standard redox potential E° between 0 V and 1.2 V; and operatively linked thereto
b) an analyzing unit configured for applying a voltage at the electrode suitable to oxidize the metal nanoparticle, and configured for measuring the detectable signal produced by the detection agent when detecting the at least one analyte; and optionally
c) an evaluating unit.

**11.** The device according to claim 10, wherein the device is adapted to perform the method according to any one of claims 1-9.

**12.** The device according to claim 10 or 11, wherein the evaluating unit is configured to determine the amount of the analyte based on the detectable signal measured by the analyzing unit.

**13.** The device according to any of claims 10-12, wherein the amount of the analyte is determined by comparing the detectable signal measured by the analyzing unit to a reference.

**14.** The device according to any of claims 10-13, further comprising at least one reference and/or at least one counter electrode.

**15.** Use of a metal nanoparticle with a standard redox potential E° between 0 V and 1.2 V as a label in electrochemical detection of an analyte by pulse voltammetry measurement, wherein the use is in a method as defined in claims 1-9.

**Patentansprüche**

1. Verfahren zum Nachweisen mindestens eines Analyten durch elektrochemischen Nachweis, umfassend:

   (i) Inkontaktbringen einer Fluidprobe, die vermutlich den mindestens einen Analyten umfasst, mit der Oberfläche einer Elektrode, die mindestens ein Bindungsmittel umfasst, das an den Analyten binden kann;
   (ii) Inkontaktbringen der Fluidprobe, die vermutlich den mindestens einen Analyten umfasst, mit mindestens einem Nachweismittel, wobei das mindestens eine Nachweismittel mindestens ein weiteres Bindungsmittel, das an den Analyten binden kann, und eine Markierung umfasst, wobei die Markierung ein Metallnanopartikel mit einem Standardredoxpotenzial E° zwischen 0 V und 1,2 V umfasst;
   (iii) Bilden eines Nachweiskomplexes auf der Oberfläche der Elektrode, der mindestens das mindestens eine Bindungsmittel, das mindestens eine Nachweismittel und den Analyten umfasst;
   (iv) Ausfällen mindestens eines Teils der Markierung auf die Elektrodenoberfläche; und
   (v) Nachweisen des mindestens einen Analyten durch elektrochemischen Nachweis;
   wobei das Verfahren einen Schritt des Auflösens mindestens eines Teils der Markierung durch Oxidation vor dem Ausfällen mindestens eines Teils der Markierung in Schritt (iv) umfasst, und
   wobei der Schritt des Auflösens mindestens eines Teils der Markierung durch Oxidation das Anlegen einer Spannung an die Elektrode, die zum Oxidieren des Metallnanopartikels geeignet ist, umfasst, wobei ein positives Potenzial, das höher ist als das Oxidationspotenzial des jeweiligen Metallnanopartikels, als diese Spannung angelegt wird, wobei die angelegte Spannung zur direkten Oxidation des Metallnanopartikels führt.

2. Verfahren nach Anspruch 1, ferner umfassend:
   einen Schritt des Bildens eines Analytkomplexes auf der Oberfläche der Elektrode, der das mindestens eine Bindungsmittel und den Analyten umfasst, vor oder gleichzeitig mit Schritt (iii) des Bildens des Nachweiskomplexes.

3. Verfahren nach Anspruch 1 oder 2, wobei die Markierung ein Metallnanopartikel von Silber oder Legierungen davon; speziell ein Silbernanopartikel oder ein Metallnanopartikel, das eine Silber/Goldlegierung umfasst oder daraus besteht; noch spezieller ein Citrat-überkapptes oder Citrat-stabilisiertes Silbernanopartikel umfasst oder daraus besteht.

4. Verfahren nach Anspruch 3, wobei das positive Potenzial als eine Spannung im Bereich von 1,0 bis 1,5 V angelegt wird.

5. Verfahren nach einem der vorstehenden Ansprüche, wobei das mindestens eine Bindungsmittel auf der Oberfläche der Elektrode, die der Fluidprobe ausgesetzt werden kann, vorzugsweise über einen Linker immobilisiert wird; vorzugsweise wobei das mindestens eine Bindungsmittel und/oder das mindestens eine weitere Bindungsmittel aus Antikörpern und Fragmenten davon, Nukleinsäuren, Aptameren, Peptidnukleinsäuren (PNAs), Rezeptor- oder Ligandenproteinen oder -peptiden und Enzymen ausgewählt sind/ist; stärker bevorzugt das Bindungsmittel und/oder das weitere Bindungsmittel einen Antikörper, noch stärker bevorzugt ein IgG umfassen/umfasst.

6. Verfahren nach einem der vorstehenden Ansprüche, wobei das Nachweismittel in einem trockenen Zustand gelagert und nach der Zugabe der Fluidprobe löslich gemacht wird.

7. Verfahren nach einem der vorstehenden Ansprüche, wobei der elektrochemische Nachweis in Schritt (v) Pulsvoltammetriemessungen, insbesondere Differenzialpulsvoltammetrie-(DPV-)Messung umfasst.

8. Verfahren nach einem der vorstehenden Ansprüche, wobei das Verfahren das Anlegen eines negativen Potenzials nach dem Ausfällen in Schritt (iv) umfasst.

9. Verfahren nach einem der vorstehenden Ansprüche 1 bis 8, wobei das Ausfällen in Schritt (iv) das Inkontaktbringen eines Gegenions mit dem gelösten Teil der Markierung umfasst, um das Bilden eines unlöslichen Salzes zu ermöglichen, das aus der Lösung ausfällt.

10. Vorrichtung zum Nachweis mindestens eines Analyten durch elektrochemischen Nachweis, umfassend:

    a) mindestens eine Arbeitselektrode, wobei die Oberfläche der Arbeitselektrode mindestens einen Linker zum Koppeln des mindestens einen Bindungsmittels umfasst und wobei die Arbeitselektrode dafür ausgebildet ist, die Bildung eines Nachweiskomplexes, der mindestens ein Nachweismittel umfasst, zu ermöglichen, wobei das mindestens eine Bindungsmittel und der Analyt auf der Oberfläche der Elektrode einer Fluidprobe ausgesetzt werden können, wobei das mindestens eine Nachweismittel mindestens ein weiteres Bindungsmittel und eine Markierung umfasst, wobei die Markierung ein Metallnanopartikel mit einem Standardredoxpotenzial E° zwischen 0 V und 1,2 V umfasst; und operativ damit verknüpft

b) eine Analyseeinheit, die zum Anlegen einer Spannung an die Elektrode, die zum Oxidieren des Metallnanopartikels geeignet ist, ausgebildet ist und zum Messen des nachweisbaren Signals, das von dem Nachweismittel beim Nachweisen des mindestens einen Analyten erzeugt wird, ausgebildet ist; und gegebenenfalls

c) eine Auswerteeinheit.

11. Vorrichtung nach Anspruch 10, wobei die Vorrichtung dafür ausgelegt ist, das Verfahren nach einem der Ansprüche 1-9 auszuführen.

12. Vorrichtung nach Anspruch 10 oder 11, wobei die Auswerteeinheit dafür ausgebildet ist, die Menge des Analyten basierend auf dem nachweisbaren Signal, das von der Analyseeinheit gemessen wurde, zu bestimmen.

13. Vorrichtung nach einem der Ansprüche 10-12, wobei die Menge des Analyten durch Vergleichen des nachweisbaren Signals, das von der Analyseeinheit gemessen wurde, mit einer Referenz bestimmt wird.

14. Vorrichtung nach einem der Ansprüche 10-13, ferner umfassend mindestens eine Referenz- und/oder mindestens eine Gegenelektrode.

15. Verwendung eines Metallnanopartikels mit einem Standardredoxpotenzial E° zwischen 0 V und 1,2 V als eine Markierung bei elektrochemischem Nachweis eines Analyten durch Pulsvoltammetriemessung, wobei die Verwendung in einem wie in den Ansprüchen 1-9 definierten Verfahren erfolgt.

**Revendications**

1. Procédé de détection d'au moins un analyte par détection électrochimique, comprenant :

(i) la mise en contact d'un échantillon de fluide suspecté de comprendre l'au moins un analyte avec la surface d'une électrode comprenant au moins un agent de liaison capable de se lier à l'analyte ;
(ii) la mise en contact de l'échantillon de fluide suspecté de comprendre l'au moins un analyte avec au moins un agent de détection, l'au moins un agent de détection comprenant au moins un agent de liaison supplémentaire capable de se lier à l'analyte et un marqueur, le marqueur comprenant une nanoparticule métallique avec un potentiel redox standard E° entre 0 V et 1,2 V ;
(iii) la formation d'un complexe de détection sur la surface de l'électrode comprenant au moins l'au moins un agent de liaison, l'au moins un agent de détection et l'analyte ;
(iv) la précipitation d'au moins une partie du marqueur sur la surface de l'électrode ; et
(v) la détection de l'au moins un analyte par détection électrochimique ;
dans lequel le procédé comprend une étape de dissolution d'au moins une partie du marqueur par oxydation avant la précipitation d'au moins une partie du marqueur de l'étape (iv), et
dans lequel l'étape de dissolution d'au moins une partie du marqueur par oxydation comprend l'application d'une tension au niveau de l'électrode appropriée pour oxyder la nanoparticule métallique, dans lequel un potentiel positif supérieur au potentiel d'oxydation de la nanoparticule métallique respective est appliqué en tant que cette tension, dans lequel la tension appliquée résulte en une oxydation directe de la nanoparticule métallique.

2. Procédé selon la revendication 1, comprenant en outre :
une étape de formation d'un complexe d'analyte sur la surface de l'électrode comprenant l'au moins un agent de liaison et l'analyte, avant ou simultanément à l'étape (iii) de formation du complexe de détection.

3. Procédé selon la revendication 1 ou 2, dans lequel le marqueur comprend ou est constitué d'une nanoparticule métallique d'argent ou d'alliages de celui-ci ; spécifiquement une nanoparticule d'argent, ou une nanoparticule métallique comprenant ou constituée d'un alliage argent/or ; plus spécifiquement une nanoparticule d'argent recouverte de citrate ou stabilisée au citrate.

4. Procédé selon la revendication 3, dans lequel le potentiel positif est appliqué en tant que tension dans la plage de 1,0 à 1,5 V.

**5.** Procédé selon l'une quelconque des revendications précédentes, dans lequel l'au moins un agent de liaison est immobilisé sur la surface de l'électrode pouvant être exposée à l'échantillon de fluide, de préférence par l'intermédiaire d'un lieur ; de préférence, dans lequel l'au moins un agent de liaison et/ou l'au moins un agent de liaison supplémentaire sont choisis parmi les anticorps et les fragments de ceux-ci, les acides nucléiques, les aptamères, les acides nucléiques peptidiques (ANP), les protéines ou peptides de récepteurs ou de ligands et les enzymes ; plus préférablement l'agent de liaison et/ou l'agent de liaison supplémentaire comprend un anticorps, encore plus préférablement une IgG.

**6.** Procédé selon l'une quelconque des revendications précédentes, dans lequel l'agent de détection est stocké à l'état sec et solubilisé lors de l'ajout de l'échantillon de fluide.

**7.** Procédé selon l'une quelconque des revendications précédentes, dans lequel la détection électrochimique de l'étape (v) comprend des mesures de voltampérométrie impulsionnelle, en particulier une mesure de voltampérométrie impulsionnelle différentielle (DPV).

**8.** Procédé selon l'une quelconque des revendications précédentes, dans lequel le procédé comprend l'application d'un potentiel négatif après la précipitation de l'étape (iv).

**9.** Procédé selon l'une quelconque des revendications 1 à 8 précédentes, dans lequel la précipitation de l'étape (iv) comprend la mise en contact d'un contre-ion avec la partie dissoute du marqueur pour permettre la formation d'un sel insoluble qui précipite de la solution.

**10.** Dispositif de détection d'au moins un analyte par détection électrochimique, comprenant :

a) au moins une électrode de travail, la surface de l'électrode de travail comprenant au moins un lieur pour le couplage de l'au moins un agent de liaison, et l'électrode de travail étant conçue pour permettre la formation d'un complexe de détection comprenant au moins un agent de détection, l'au moins un agent de liaison et l'analyte sur la surface de l'électrode pouvant être exposés à un échantillon de fluide, l'au moins un agent de détection comprenant au moins un agent de liaison supplémentaire et un marqueur, le marqueur comprenant une nanoparticule métallique avec un potentiel redox standard E° entre 0 V et 1,2 V ; et liée de manière fonctionnelle à celui-ci

b) une unité d'analyse configurée pour appliquer une tension au niveau de l'électrode appropriée pour oxyder la nanoparticule métallique, et configurée pour mesurer le signal détectable produit par l'agent de détection lors de la détection de l'au moins un analyte ; et éventuellement

c) une unité d'évaluation.

**11.** Dispositif selon la revendication 10, dans lequel le dispositif est adapté pour réaliser le procédé selon l'une quelconque des revendications 1 à 9.

**12.** Dispositif selon la revendication 10 ou 11, dans lequel l'unité d'évaluation est configurée pour déterminer la quantité de l'analyte en se basant sur le signal détectable mesuré par l'unité d'analyse.

**13.** Dispositif selon l'une quelconque des revendications 10 à 12, dans lequel la quantité de l'analyte est déterminée en comparant le signal détectable mesuré par l'unité d'analyse à une référence.

**14.** Dispositif selon l'une quelconque des revendications 10 à 13, comprenant en outre au moins une référence et/ou au moins une contre-électrode.

**15.** Utilisation d'une nanoparticule métallique avec un potentiel redox standard E° entre 0 V et 1,2 V en tant que marqueur dans la détection électrochimique d'un analyte par mesure de voltampérométrie impulsionnelle, dans laquelle l'utilisation est dans un procédé tel que défini dans les revendications 1 à 9.

Figures

Figure 1

Figure 2

Figure 3

Figure 4

Figure 5

Figure 6

**Figure 7**

**Figure 8**

A

B

Figure 9

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2016997 A1
- EP 3610947 A1
- WO 2017216339 A1

**Non-patent literature cited in the description**

- **HAO N ; LI H ; LONG Y ; ZHANG L ; ZHAO X ; XU D ; CHEN H-Y**. An electrochemical im-munosensing method based on silver nanoparticles. *Journal of Electroanalytical Chemistry*, 2011, vol. 656 (1-2), 50-54
- **KHRISTUNOVA Y et al.** Preparation and Investigation of Silver Nanoparticle-Antibody Bioconjugates for Electrochemical Immunoassay of Tick-Borne Encephalitis. *SENSORS*, 2019, vol. 19 (9), 2103
- **LA ESCOSURA-MUÑIZ A DE ; PAROLO C ; MARAN F ; MEKOÇI A**. Size-dependent direct electrochemical detection of gold nanoparticles: application in magnetoimmunoassays. *Nanoscale*, 2011, vol. 3 (8), 3350-3356
- **POLLOK NE ; RABIN C ; WALGAMA CT ; SMITH L ; RICHARDS I ; CROOKS RM**. Electrochemical Detection of NT-proBNP Using a Metalloimmunoassay on a Paper Electrode Platform. *ACS Sens*, 2020, vol. 5, 853-860
- **PUMERA M ; ALDAVERT M ; MILLS C ; MERKOÇI A ; ALEGRET S**. Direct voltammetric determination of gold nanoparticles using graphite-epoxy composite electrode. *Electrochimica Acta*, 2005, vol. 50 (18), 3702-3707
- **SZYMANSKI M ; TURNER AF ; PORTER R**. Electrochemical Dissolution of Silver Nanoparticles and Its Application in Metalloimmunoassay. *Electroanalysis*, 2010, vol. 22 (2), 191-198
- **SZYMANSKI MS ; PORTER RA**. Preparation and quality control of silver nanoparticle-antibody conjugate for use in electrochemical immunoassays. *Journal of immunological methods*, 2013, vol. 387 (1-2), 262-269
- **TING BP ; ZHANG J ; GAO Z ; YING JY**. A DNA biosensor based on the detection of dox-orubicin-conjugated Ag nanoparticle labels using solid-state voltammetry. *Biosensors & bi-oelectronics*, 2009, vol. 25 (2), 282-287